# EUROPEAN PATENT APPLICATION

(11) **EP 1 726 303 A1**
(43) Date of publication of application: **29.11.2006**
(21) Application number: 05727041.5
(22) Date of filing: 22.03.2005
(51) Int. Cl.: A61K 31/198, A61P 7/06

(54) **THERAPEUTIC AGENT FOR RENAL ANEMIA**

(30) Priority: 19.03.2004 JP 2004081024; 08.09.2004 JP 2004261156; 02.11.2004 JP 2004319333
(71) Applicant: Ajinomoto Co., Inc., Tokyo 104-8315 (JP)
(72) Inventor: TARUMOTO, Takahisa, Minamimatsuura-gun, Nagasaki 8574211 (JP); IMAGAWA, Shigehiko, Ibaraki 3050056 (JP)
(74) Representative: Nicholls, Kathryn Margaret
(86) International application number: PCT/JP2005/005117
(87) International publication number: WO 2005/089743

(57) **Abstract**

There is provided an agent for preventing or treating renal anemia, which contains arginine as an active ingredient. This agent for preventing or treating renal anemia can be easily administered and the administration can be controlled at home to obtain excellent effect in preventing or treating renal anemia.

## Description

### Technical Field

The present invention relates to an agent for preventing or treating renal anemia and also a method for preventing or treating renal anemia patients.

### Background of the Invention

Patients suffering from chronic renal failure will be anemic as the renal function of them is weakened. The anemia is called "renal anemia" because it starts in the second stage (functional renal disease) in the staging of chronic renal failure and this anemia crisis is caused in almost the all cases in the third stage (chronic renal failure). A blood transfusion was given to patients suffering from renal anemia for improving the anemia in old times and the treatment with a recombinant erythropoietin preparation (rHuEPO preparation) is common at present.

However, it was pointed out that the treatment with rHuEPO preparation requires seeing a doctor regularly and that the patient feels a pain during the subcutaneous injection. Under these circumstances, it is demanded to develop a side effect-free agent for preventing or treating renal anemia, which has a further improved clinical effect and can be easily taken and the taking of which can be controlled at home. In addition, it is also demanded to develop an agent for preventing or treating renal anemia, which does not provoke an increase in the amount of guanidino compounds (methylguanidine and guanidinosuccinic acid) and blood urea nitrogen (BUN) because the increase in amount of the guanidino compounds which are uremic toxins and the increase of BUN due to an excessive intake of protein further damage the renal function of cases of chronic renal failure, particularly renal failure in predialysis.

Arginine is known to be a substrate for a nitric oxide synthetase. However, it has not been reported that arginine improves patients suffering from renal anemia.

The inventors are investigating the mechanism of producing erythropoietin by using a nitric oxide synthetase inhibitor and L-arginine. It has not yet been found that L-arginine is effective on renal anemia [non-patent documents 1 and 2].
[Non-Patent Document 1] S. Imagawa et al., blood, 96, 1716-1722 (2000)
[Non-Patent Document 2] S. Imagawa et al., Kidney Int., 61, 396-404 (2002).

### Disclosure of the Invention

The object of the present invention is to provide an agent having an excellent effect for preventing or treating renal anemia; that agent can be easily taken and its administration can be controlled at home.

After intensive investigations made for the purpose of finding an excellent agent for preventing or curing renal anemia, the inventors have found that a composition containing arginine as an active ingredient has an excellent effect of preventing or curing anemia. The present invention has been completed on the basis of this finding.

Namely, the present invention provides an agent for preventing or curing renal anemia including the following embodiments:
(1) An agent for preventing or treating renal anemia, which contains arginine as an active ingredient.
(2) The agent for preventing or treating renal anemia described in (1), which is for human beings.
(3) The agent for preventing or treating renal anemia described in (1) or (2), which is for living bodies having an erythropoietin concentration in blood of at least 3 mIU/mL.
(4) The agent for preventing or treating renal anemia described in any of (1) to (3), which is administered in a dose of 0.5 to 12 g/day to adults.
(5) The agent for preventing or treating renal anemia described in any of (1) to (4), which further contains erythropoietin.
(6) The agent for preventing or treating renal anemia described in any of (1) to (5), which is given by oral administration.
(7) The agent for preventing or treating renal anemia described in any of (1) to (5), which is given by enteral administration.
(8) The agent for preventing or treating renal anemia described in any of (1) to (5), which is to be given in the course of the hemodialysis or peritoneal dialysis.
(9) The agent for preventing or treating renal anemia described in any of (1) to (8), which contains arginine and/or a physiologically acceptable salt thereof as the active ingredient.
(10) The agent for preventing or treating renal anemia described in (9), which contains free L-arginine and L-arginine monohydrochloride as the active ingredients.
(11) The agent for preventing or treating renal anemia described in any of (1) to (10), which further contains a chalybeate.
(12) A hemoglobin concentration-increasing agent, particularly an agent for increasing hemoglobin concentration of human beings, containing arginine as an active ingredient.

### Brief Description of the Drawing

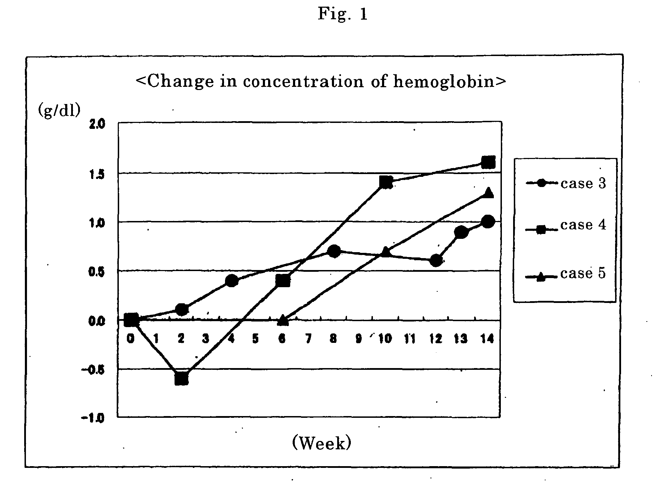

Fig. 1 shows changes in hemoglobin concentration of patients to whom arginine was administered.

### Best Mode for Carrying out the Invention

The agent for preventing or treating renal anemia of the present invention contains arginine as an active ingredient and, preferably, pharmaceutically acceptable preparation components.

Arginine used as the active ingredient in the present invention preferably includes arginine *per se* and/or physiologically acceptable salts thereof. It is more preferred to use the combination of free L-arginine and L-arginine monohydrochloride.

As for isomers of the respective active ingredients, L-, D- and DL-isomers are usable. However, L-isomers are preferred because they are natural products.

The dosage form of the agent for preventing or treating renal anemia is not particularly limited in the present invention. The agent can be given by the oral administration, and this method is preferred. The dose varies depending on the erythropoietic capacity, hemoglobin concentration which is an index of anemia and age of the patient and also on the administration method. Usually for adults, the dose is about 0.5 to 12 g, preferably about 1 to 6 g, a day.

The dose for day can be taken at once or it can be divided into several portions. In the oral administration, the dose is preferably not larger than 5 g, more preferably not larger than 3 g, each time.

On the other hand, in the enteral administration or infusion administration, the active ingredient can be administered in the form of a solution together with various nutritious sources. Although the active ingredient of the present invention can be given by means of the parenteral administration such as drip infusion or injection (intravenous administration), either oral or enteral administration is preferred because it is very efficiently absorbed through the digestive tracts. In the cases of renal anemia, those undergoing dialysis are preferably given the active ingredient of the present invention in the course of the dialysis.

The active ingredient of the present invention can be incorporated into a pharmaceutical composition together with various substances for the preparation by an ordinary method. The substances for the preparation can be suitably selected depending on the dosage form such as tablets and granules and they include, for example, excipients, diluents, additives, disintegrators, binders, coating agents, wetting agents, gliding agents, lubricants, flavoring agents, sweetening agents, solubilizers and stabilizers. Concrete examples of the substances for the preparation include magnesium carbonate, titanium dioxide, lactose, mannitol and other saccharides, talc, milk protein, starch, (crystalline) cellulose and derivatives thereof (such as hydroxypropylcellulose), animal and vegetable oils, polyethylene glycols, Carmellose calcium, and solvents such as sterilized water and monohydric or polyhydric alcohols such as glycerol. Among them, crystalline cellulose, hydroxypropylcellulose and Carmellose calcium are preferred in the present invention.

The substance used for the preparation is preferably one of the corrigents, excipients and coating agents which are not harmful to the absorption of the active ingredient through the gastrointestinal tracts.

As for the dosage form, a powder or coated grains such as coated granules are preferred from the viewpoint of difficulty of the swallowing of aged patients. It is also preferred to use corrigents with the active ingredient to realize a dosage form easy for aged patients to take.

In the present invention, the active ingredient can be used in the form of a mixture or in combination with another pharmacological ingredient (active medicinal substance). In such a case, chalybeates and rHuEPO can be mentioned as the agents that can be used together with the active ingredient of the present invention in a suitable proportion to exhibit a synergistic effect on the improvement of anemia. It is preferred to use both chalybeate and rHuEPO together. Further, vitamin C can be used together with the chalybeate for improving the absorption of iron.

A part or the whole of arginine used as the active ingredients in the present invention can be used in the form of their salts.

As described above, the agent of the present invention for preventing or treating anemia can be prepared in various medicinal preparation forms which are well known as described above or which will be developed in future, for various administration methods such as oral administration, intraperitoneal administration, percutaneous administration, subcutaneous administration, intravenous administration and inhalation administration. For preparing the medicinal preparation in various forms and containing the active ingredient of the present invention, known methods or methods which will be developed in future can be suitably selected.

As a matter of course, the agent of the present invention for preventing or treating renal anemia in the dosage form exemplified above should contain the above-described components in an amount effective for improving the anemia.

"Argi U granules" are placed on the market as an agent for treating congenital urea cycle disorder containing arginine as the active ingredient. This preparation contains an equimolar mixture of L-arginine and L-arginine monohydrochloride as the active ingredients and it can be orally administered. Therefore, this preparation is useful as an embodiment of the agent of the present invention for preventing or treating renal anemia.

The term "renal anemia" herein indicates anemia of cases of chronic renal failure. The chronic renal failure is divided into four stages (the staging of Seldin et al.), namely, the first stage (reduction in renal reserve), the second stage (functional renal disease), the third stage (chronic renal failure) and the fourth stage (uremia). The cases of renal anemia increase in number in the second stage (functional renal disease) and thereafter as the stages proceed. The underlying diseases of chronic renal failure are, for example, diabetic nephropathy, chronic glomerulonephritis, nephrosclerosis, polycystic kidney and chronic pyelonephritis. Further, it is preferred that the preventing or treating agent of the present invention is used for patients having an erythropoietin concentration in the blood of at least 3 mIU/mL or at least 4 mIU/mL. The erythropoietin concentration in the blood of the patients can be determined by determining erythropoietin concentration in the plasma by RIA or ELISA determination method.

The agent of the present invention for treating or preventing renal anemia is capable of preventing or treating anemia of patients with renal anemia and also improving the clinical symptoms caused by anemia such as lowering of will to work, fatigability, shortness of breath, lightheadedness and palpitation. In addition, when this agent is prepared for the oral administration, it can be easily taken at home everyday without pain caused by the subcutaneous injection and also without regularly seeing a doctor and, therefore, the effect of improving anemia can be surely obtained. Another advantage of the agent is that substantially no side effect is caused by the administration and the patients can be treated without fear of worsening of the renal function.

With the agent of the present invention, the control of symptoms caused by anemia is made possible without administration of rHuEPO preparation. When this agent is used in combination with rHuEPO preparation, the interval of the administration of the rHuEPO preparation (once a week) can be elongated. In addition, in this case, the continuous administration is possible without fear of an increase in the blood pressure of the patient due to the rapid hematogenesis because hematocrit value and hemoglobin concentration are slowly increased. Another merit of this agent is that because it can be stored at room temperature, it can be extremely easily kept in hospitals or at home. Although an anti-erythropoietin antibody is formed sometimes and pure red cell aplasia (in patients suffering from pure red cell aplasia, the red cell formation in only erythrocytic system in the 3 bone marrow blood systems is selectively lowered or the formation is stopped, leading to anemia) might be caused by the administration of rHuEPO, the present invention is free of such a side effect.

According to the present invention, an agent for increasing hemoglobin concentration, in particular, an agent for increasing human hemoglobin concentration, can be provided by using arginine.

According to the present invention, the prevention or treatment of anemia of patients suffering from renal anemia is made possible, and the clinical symptoms caused by anemia such as lowering of will to work, fatigability, shortness of breath, lightheadedness and palpitation can be improved. In addition, according to the present invention, this agent can be easily taken at home everyday without pain caused by the subcutaneous injection and also without regularly seeing a doctor and, therefore, the effect of improving anemia can be surely obtained. Another advantage of the agent is that side effects are scarcely caused by the administration and the patients can be treated without fear of worsening of the renal function.

The following Examples will further specifically illustrate the present invention, which by no means limit the invention.

### Example 1

### Clinical Test Example 1 (Effect of improving anemia of patients with renal anemia)

1.3 g of an amino acid preparation (trade name: Argi U granules, a product of Ajinomoto Pharma Co. Ltd.) (1.0 g of arginine) was orally administered in two portions everyday to each of 8 patients having an illness diagnosed as renal anemia (cases of chronic renal failure in conservative stage (predialysis) accompanying renal anemia, whose age, hemocytometric results (hemoglobin concentration) and results of general biochemical tests are shown in Table 1). The administration period was at the longest 18 weeks. The amount of each of hemoglobin, BUN (blood urea nitrogen), Cre (creatinine), Fe and erythropoietin in blood was determined by methods described below.

Hemoglobin was determined with an automatic blood cell analysis device.

BUN was determined by urease method / ammonia elimination method.

Cre was determined by an enzymatic method (sarcosine oxidase· POD method).

Fe was determined by nitroso-PSAP (5-(N-propyl-N-sulfopropylamino) phenol) method.

Erythropoietin was determined by ELISA method.

The hemoglobin concentration of each case determined before and after the administration of the amino acid preparation is shown in Table 2.

For typical three patients, a change in hemoglobin concentration was given as Δ Hb value (amount of change of hemoglobin) calculated by deducting the hemoglobin concentration before the administration of the amino acid preparation from the hemoglobin concentration after the administration thereof in Fig. 1.

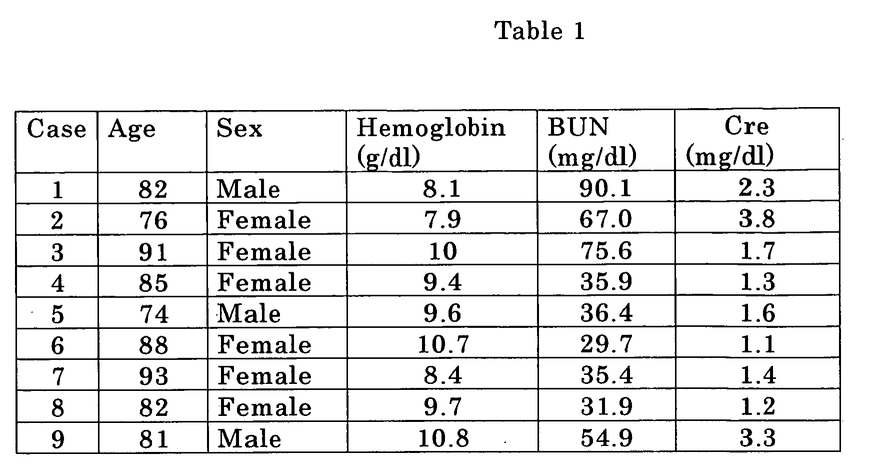

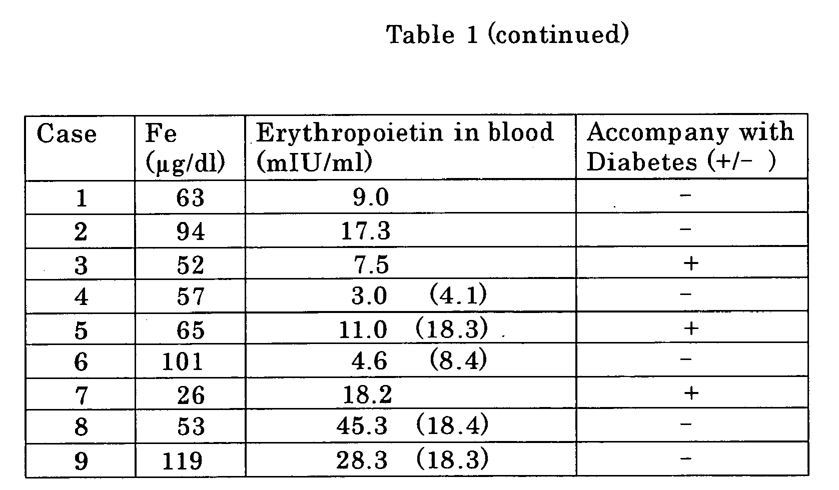

In Table 1, the values of Erythropoietin in blood in cases 4 to 6 and 8 to 9 are those obtained by the second determination, and those obtained by the first determination are shown in the parentheses.

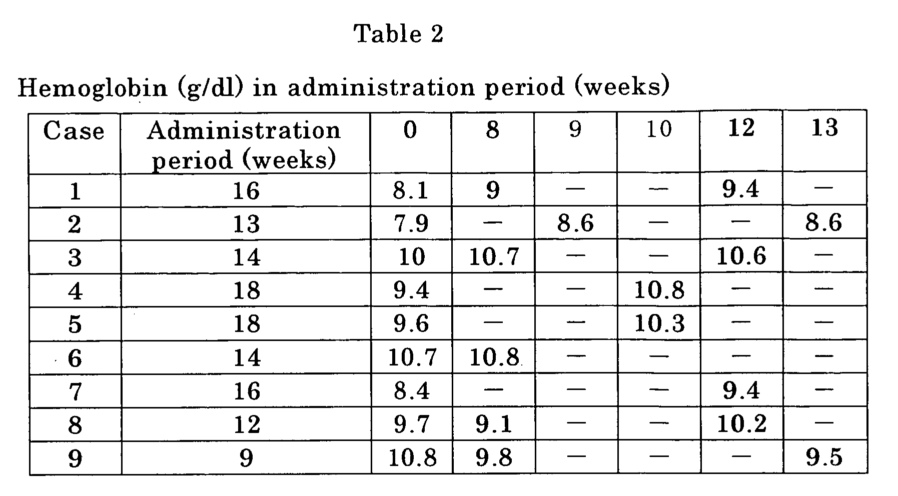

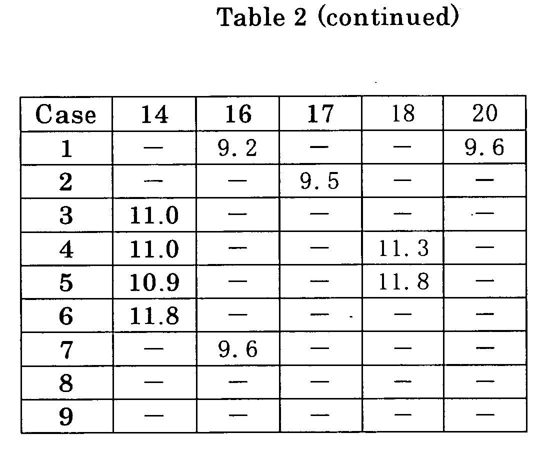

The results are summarized in Table 3.

**Table 3**

| Case | Maximum hemoglobin (g/dl) | Minimum BUN (mg/dl) | Minimum Cre (mg/dl) | Maximum erythropoietin in blood (mIU/ml) |
|---|---|---|---|---|
| 1 | 9.6 | 62.6 | 1.9 | 11.5 |
| 2 | 9.5 | 58.6 | 2.7 | 19.6 |
| 3 | 11.0 | 67.8 | 1.5 | 8.7 |
| 4 | 11.3 | 13.7 | 0.8 | 7.7 |
| 5 | 11.8 | 28.4 | 1.5 | 18.1 |
| 6 | 11.8 | 19.7 | 0.8 | 20.9 |
| 7 | 9.8 | 50.9 | 1.4 | 12.6 |
| 8 | 10.2 | 34.8 | 1.3 | 34.0 |
| 9 | 10.6 | 40.0 | 3.1 | 22.3 |

In cases 1 to 7 shown in Table 3, Hb was increased by 1.0 to 2.2 g/dL, and the results were judged to be effective. In the cases wherein the effect of arginine was recognized, erythropoietin concentration in blood was at least 3 mIU/mL. On the other hand, in cases 8 and 9, Hb was decreased by 0.6 to 1.3 g/dL and the results were judged to be ineffective. In the comparison of Epo productivity before the administration of L-arginine in the effective cases with that in the ineffective cases, it is understood that Epo production was low, i.e., 3.0 to 18.2 mU/mL while Hb value was low in the former. On the other hand, Epo productivity was kept at 28.3 to 45.3 mU/mL while Hb was low in the ineffective case. In 2 cases in the 7 effective cases, anemia became worse after the discontinuance of L-arginine administration, but in other 3 cases, Hb was not lowered. In the effective cases, the recovery of the renal function was recognized in cases 1, 2, 4 and 6. These cases coincided with the cases in which Hb was not lowered after the discontinuance of L-arginine administration. On the other hand, as for Epo concentration in blood, it was recognized that Ep productivity was increased by the administration of L-arginine in all the effective cases. However, in the two ineffective cases, the Epo productivity was lowered. No evident side effect was recognized in the 9 administration cases.

From the above-described results, it is considered that by the administration of L-arginine, the plasma L-arginine concentration is relatively increased and that lead to cancel of suppression on NO (nitric oxide)· cGMP production. As a result, it is suggested that inhibition of increase in GATA binding activity lead to cancel the suppression of the Epo promoter activity and therefore to recover the Epo productivity, thereby improving the renal anemia. However, since it was recognized that the renal function was improved by the administration of L-arginine in 4 cases in the 7 effective cases, this fact suggested the possibility of the improvement in the renal anemia by the recovery of the renal function independent of Epo production. After the further administration of 2.6 g/day to the patients for 1 month, Hb was increased from 10.7 g/dl to 11.5 g/dl, while no obvious side effect was recognized.

EPO concentration in blood was at the highest about 20 mU/mL after the administration of L-arginine, while it was increased to about 130 mU/mL in the conventional treatment with rHuEPO preparation. Namely, EPO concentration in blood in the former case was less than 1/6 as compared with that in the latter case. Thus, the therapeutic method of the present invention is free from side effects and safer than the conventional therapeutic method wherein rHuEPO preparation is used.

Thus, the effect for preventing or treating renal anemia can be expected when arginine is orally administered to patients with renal anemia. Other advantages of the agent are that it is free of side effects and that the administration of this agent can be easily controlled at home.

## Claims

1. An agent for preventing or treating renal anemia, which contains arginine as an active ingredient.

2. The agent for preventing or treating renal anemia of claim 1, which is for human beings.

3. The agent for preventing or treating renal anemia of claim 1 or 2, which is for living bodies having an erythropoietin concentration in blood of at least 3 mIU/mL.

4. The agent for preventing or treating renal anemia of any one of claims 1 to 3, which is administered in a dose of 0.5 to 12 g/day to adults.

5. The agent for preventing or treating renal anemia of any one of claims 1 to 4, which further contains erythropoietin.

6. The agent for preventing or treating renal anemia of any one of claims 1 to 5, which is given by oral administration.

7. The agent for preventing or treating renal anemia of any one of claims 1 to 5, which is given by enteral administration.

8. The agent for preventing or treating renal anemia of any one of claims 1 to 5, which is to be given in the course of the hemodialysis or peritoneal dialysis.

9. The agent for preventing or treating renal anemia of any one of claims 1 to 8, which contains arginine and/or a physiologically acceptable salt thereof as the active ingredient.

10. The agent for preventing or treating renal anemia of claim 9, which contains free L-arginine and L-arginine monohydrochloride as the active ingredients.

11. The agent for preventing or treating renal anemia of any one of claims 1 to 10, which further contains a chalybeate.

12. A hemoglobin concentration-increasing agent for human beings, containing arginine as an active ingredient.

13. A method for preventing or treating renal anemia, which comprises administering arginine and erythropoietin to a subject.
